# EUROPEAN PATENT APPLICATION

(11) **EP 2 722 079 A1**
(43) Date of publication of application: **23.04.2014**
(21) Application number: 12189460.4
(22) Date of filing: 22.10.2012
(51) Int. Cl.: A63B 24/00, A61B 5/0488

(54) **Muscle activity training facility for the lower body of a user**

(71) Applicant: Chen, Paul, Vancouver, BC V7A 1S5 (CA)
(72) Inventor: Chen, Paul, Vancouver, BC V7A 1S5 (CA)
(74) Representative: Beck & Rössig European Patent Attorneys

(57) **Abstract**

A muscle activity training facility includes a monitor (12) coupling to a processor (11) and having a screen (15) which includes two areas (16, 17), and two figures (18, 19) in one of the areas (16) for indicating the standard operations of the muscles in front and rear portions of the user, and two further figures (20, 21) in the other area (17) for indicating the muscles in the front and the rear portions of the user, a carrier (30) is attached to a lower body (80) of the user, and a number of electrodes (40-49) are attached to the carrier (30) and coupled to the processor (11) for detecting and showing the detected activities of the muscles of the user in the other figures (20, 21) in the other area (17) of the screen (15).

## Description

The invention relates to a muscle activity training facility for detecting or training the muscles in the lower body of the user.

Typical muscle activity training facilities comprise one or more sensors or detectors for detecting the muscle activities of muscles of the user.

However, the sensors or detectors are attached to the hairy portions of the user with tapes or adhesives and may have a good chance to be disengaged from the user after use.

The invention is to provide a muscle activity training facility for attaching to the lower body of the user and for detecting the muscle exercises or activities in the lower body of the user.
Figs. 1, 2 are plan views of a muscle activity training facility;
Fig. 3 is a lower perspective view of a carrier;
Figs. 4, 5 are partial plan views of a front and a rear portion of a user; and
Figs. 6, 7, 8, 9 are plan views of the screen of the facility.

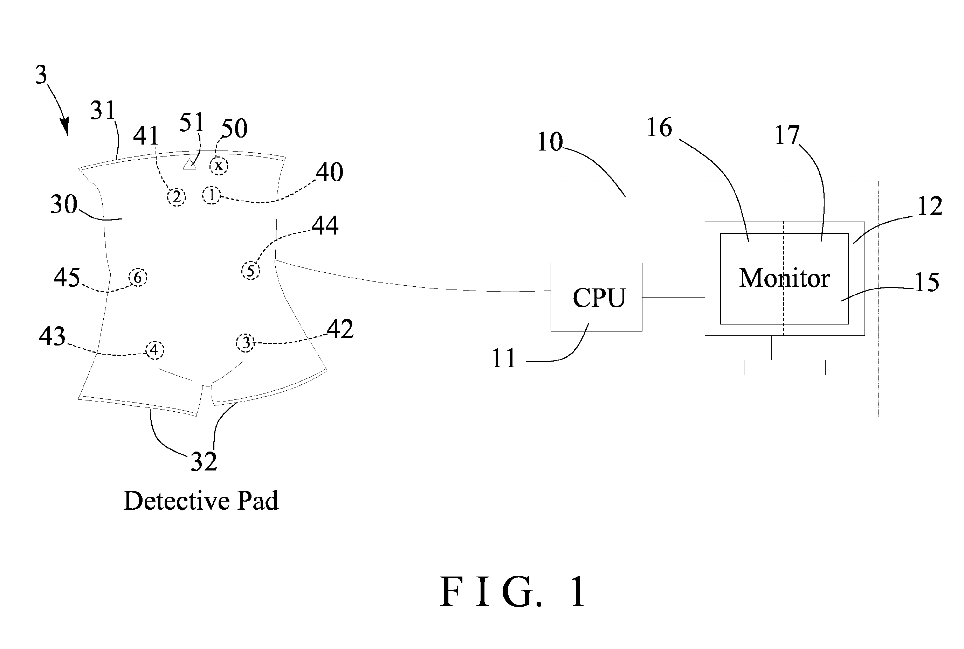

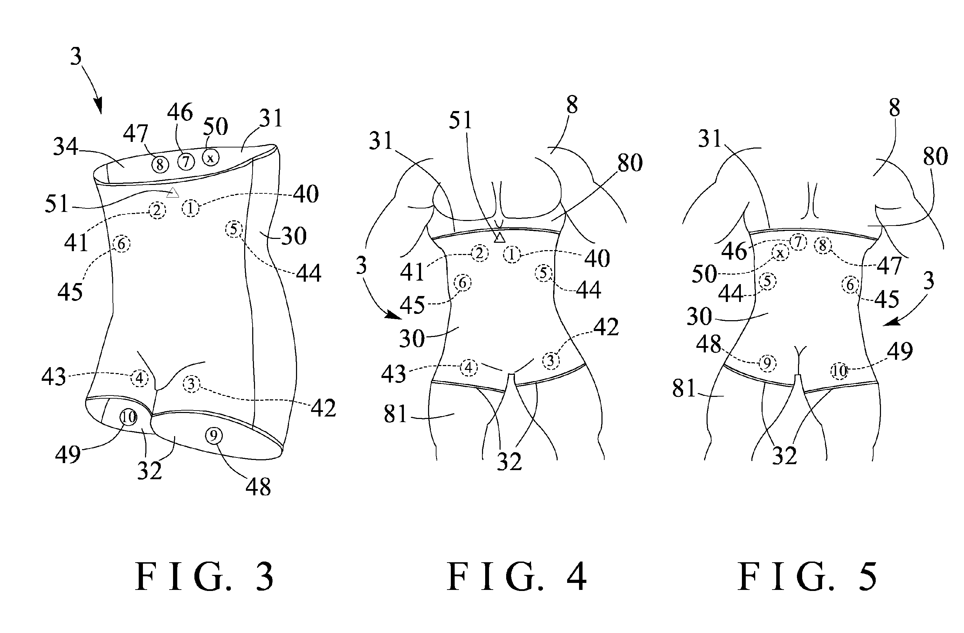

Referring to Fig. 1, a muscle activity training facility comprises a computer 10 including a processer 11, a monitor 12 coupled to the processor 11 and having a screen 15 for showing data or information, such as the front and the rear views of the user 8 (Figs. 4-5) or the activities or exercises of the user 8. An adaptor 3 (Figs. 1, 3-5) includes a cloth or carrier 30 for firmly attaching to the lower body 80 of the user 8 without disengaging from the user 8 even when exercising, the carrier 30 includes a chamber 31 (Fig. 3) for receiving the lower body 80 of the user 8, two lower openings 32 for engaging with the legs 81.

A number of electrodes 40-49 (Figs. 1, 3-5) are attached to the inner portion 34 of the carrier 30 (Fig. 3) at selected positions, for example, the electrodes 40-43 are disposed at the front portion of the carrier 30 for engaging with the abdominal or legs of the lower body 80, the electrodes 44, 45 are engaged onto the side positions of the lower body 80 of the user 8, and the other electrodes 46-49 are disposed to engage with the back portion, such as the middle and back portion and the upper legs of the lower body 80. Another electrode 50, such as a G-sensor 50 (Figs. 1, 3, 5) is attached to the carrier 30 at the other selected positions, such as the upper or back portion of the carrier 30 corresponding to the back portion of the user 8, such that the electrodes 40-50 may sense or detect the muscle activities of the user 8, and are coupled to the processor 11.

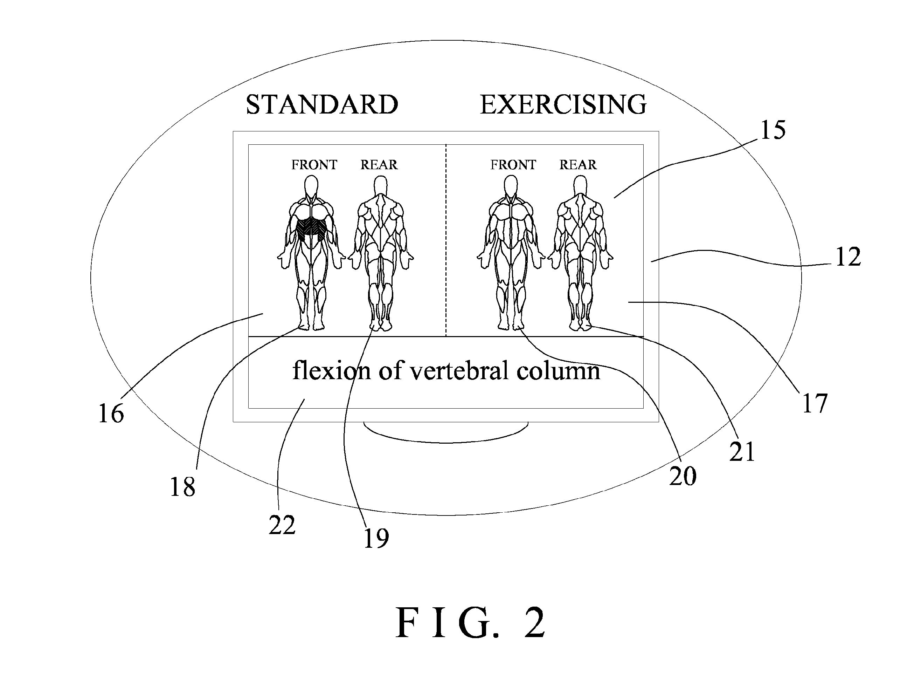

The adaptor 3 includes one or more pointers 51 (Figs. 1, 3-4) for aligning with selected portions of the user 8, such as the manubrium, the sternum, or the xiphoid process of the user 8 (Fig. 4), etc. for aligning the electrodes 40-50 with the selected portions of the user 8. The screen 15 includes a left or first area 16 (Figs. 2, 6-9) having two figures 18, 19 for showing the standard or correct activities of the front and the rear portions of the user 8 that have been recorded or stored in the computer 10 and shown by shaded lines, different colors, the darkness, the brightness or the like. The right or second area 17 of the screen 15 includes two figures 20, 21 shown by shaded lines, different colors, the darkness, the brightness or the like for showing the exercising of the muscles in the front and the rear portions of the user 8 that are detected by the electrodes 40-50 or processed by the processor 11 of the computer 10.

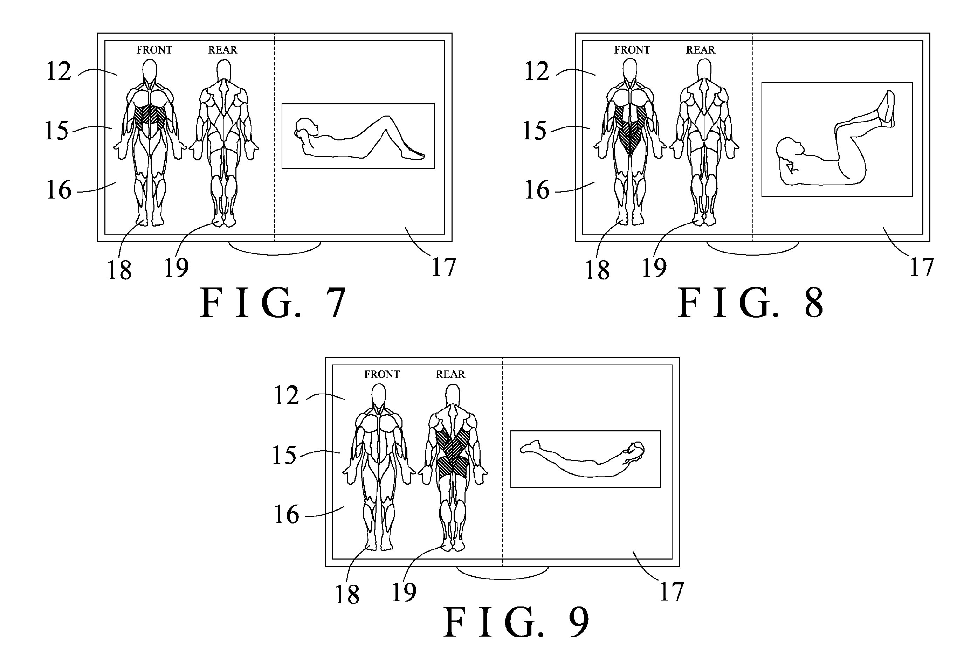

The right or second area 17 of the screen 15 may be used to show the operation of the user 8, such as such as the flexion of the vertebral column (Fig. 7), the flexion of thigh or knee bends (Fig. 8), the extension of the vertebral column and the extension of the thigh of the user 8 (Fig. 9). The left area 16 of the screen 15 may show the muscles of the user 8 are properly or correctly operated and recorded or stored in the computer 10 as the standard or correct activities or operations. The activities that have not been shown in Figs. 7-9 may also be recorded or stored in the computer 10.

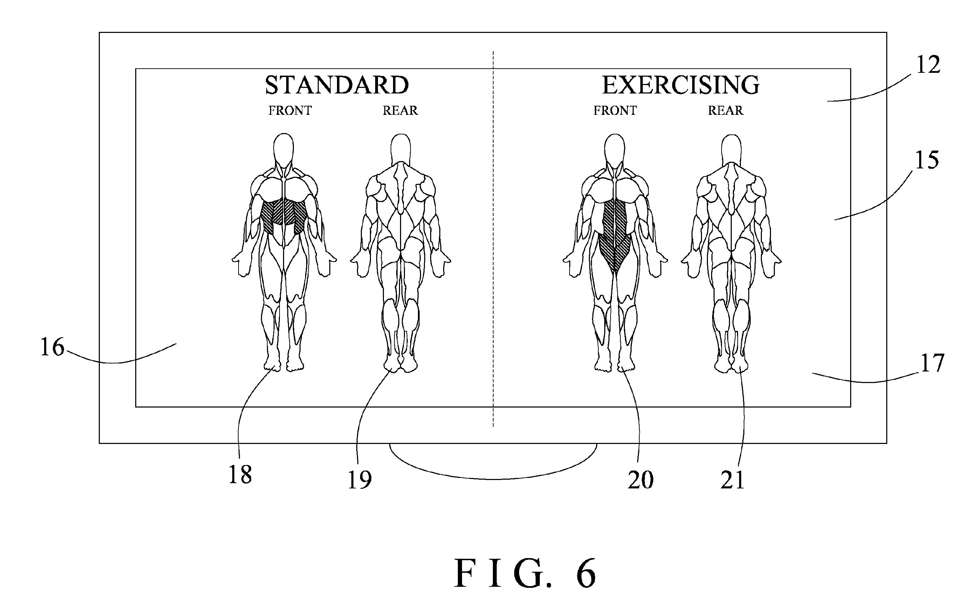

In operation, the carrier 30 may be attached to the lower body 80 of the user 8 by aligning the pointers 51 with the selected positions of the user 8, and the user may then select the required activity or exercise, such as the flexion of the vertebral column, which will be shown in the selected area 22 of the screen 15, and shown in the right or second area 17 of the screen 15 (Figs. 7-9). When the user 8 is conducting the selected exercises (Figs. 2, 6), the figures 20, 21 in the right or second area 17 of the screen 15 may be shown by shaded lines, different colors, the darkness, the brightness or the like for indicating the activities or exercises of the muscles in the lower body 80 of the user 8. The user 8 may then compare the figures 20, 21; 18, 19 to determine the difference between the muscles in order to correct the activity or exercise operation of the user 8.

## Claims

1. A muscle activity training facility comprising a processor (11), a monitor (12) coupled to the processor (11) and having a screen (15), **characterized in that**:
the screen (15) includes a first area (16) and a second area (17), two first figures (18, 19) in the first area (16) for indicating standard operations of muscles in front and rear portions of a user, and two second figures (20, 21) in the second area (17) for indicating operations of muscles in the front and the rear portions of the user, a carrier (30) attached to a lower body (80) of the user, and a plurality of electrodes (40-49) attached to the carrier (30) and coupled to the processor (11).

2. A muscle activity training facility as claimed in claim 1, wherein the carrier (30) includes a chamber (31) for receiving the lower body (80) of the user, and two lower openings (32) for engaging with legs (81) of the user.

3. A muscle activity training facility as claimed in claim 1 or 2, wherein the carrier (30) includes a G-sensor (50).

4. A muscle activity training facility as claimed in one of claims 1 to 3, wherein the monitor (12) includes a selected area (22) of the screen (15).

5. A muscle activity training facility as claimed in one of claims 1 to 4, wherein the carrier (30) includes at least one pointer (51) for aligning with a reference portion of the user.

6. A muscle activity training facility as claimed in one of claims 1 to 5, wherein the carrier (30) is selected from a cloth.
